# EUROPÄISCHE PATENTANMELDUNG

(11) **EP 2 014 253 A2**
(43) Veröffentlichungstag der Anmeldung: **14.01.2009**
(21) Anmeldenummer: 08012533.9
(22) Anmeldetag: 10.07.2008
(51) Int. Cl.: A61C 9/00, A61C 13/00

(54) **Verfahren zum Darstellen von Restzahnbereichen und/oder geplanten Zahnersatzteilversorgungen sowie dazugehöriges computerlesbares Medium, Computer und Verfahren zum Versenden einer Datei**

(30) Priorität: 11.07.2007 DE 102007032291
(71) Anmelder: Institut Straumann AG, 4002 Basel (CH)
(72) Erfinder: Holzner, Stephan, 82069 Schäftlarn (DE); Weber, Gerhard, 86932 Pürgen (DE)
(74) Vertreter: Grünecker, Kinkeldey, Stockmair & Schwanhäusser Anwaltssozietät

(57) **Zusammenfassung**

Die Erfindung betrifft ein Verfahren zum Darstellen von Restzahnbereichen und/oder geplanten Zahnersatzteilversorgungen mit den Schritten: Darstellen des Restzahnbereichs und/oder der Zahnersatzteilversorgung in einem ersten grafischen Modus und Darstellen desselben Restzahnbereichs und/oder der Zahnersatzteilversorgung in einem zweiten grafischen Modus, der verschieden ist von dem ersten grafischen Modus. Weiterhin betrifft die Erfindung ein Verfahren zum Darstellen von Restzahnbereichen und/oder geplanten Zahnersatzteilversorgungen mit dem Schritt: Darstellen des Restzahnbereichs und/oder der Zahnersatzteilversorgung in einem grafischen Modus, der einen, zwei, drei, vier oder mehr der folgenden Punkte aufweist: eine photorealistische Darstellung, eine Bildauflösung von höher als 300 dpi, mindestens eine 24 bit- Farbtiefe, die Darstellung von Schattenwurf, die Darstellung von Spiegeleffekten und/oder Reflexen, die Darstellung von Transparenzen, die Darstellung von Nebeleffekten, mindestens zwei, drei, vier oder mehr simulierte Lichtquellen und farbrealistische Darstellung von verschiedenen Bereichen, wie etwa Zahnfleisch, Zahnmaterial oder Zahnersatzteilen. Ebenso betrifft die Erfindung ein dazugehöriges computerlesbares Medium, einen Computer und ein Verfahren zum Versenden einer Datei.

## Beschreibung

Die Erfindung betrifft ein Verfahren zum Darstellen von Restzahnbereichen und/oder geplanten Zahnersatzteilversorgungen.

Zur Planung von Zahnersatzteilversorgungen ist es bekannt, Daten eines Restzahnbereichs bzw. einer solchen geplanten Zahnersatzteilversorgung anzuzeigen. Die Daten des Restzahnbereichs können mit einem Scanner gewonnen worden sein, die Daten der geplanten Zahnersatzteilversorgung können ebenfalls von einem Scanner gewonnen worden oder auch durch eine Modellierung entstanden sein.

Solche Darstellungen erlauben es einem Zahntechniker beispielsweise die Daten der geplanten Zahnersatzteilversorgung zu modellieren, überprüfen, ändern und anzupassen. Hierzu sind bestimmte Darstellungsformen hilfreich, wie beispielsweise die Darstellungsform mit Falschfarben, Hilfslinien und Ähnlichem.

Aufgabe der vorliegenden Erfindung ist es, die Darstellung von Restzahnbereichen oder geplanten Zahnersatzteilversorgungen zu verbessern.

Diese Aufgabe wird gelöst mit einem Verfahren nach Anspruch 1, einem Verfahren nach Anspruch 12, einem computerlesbaren Medium nach Anspruch 18, einem Computer oder einer Mehrzahl von Computern nach Anspruch 19 oder einem Verfahren nach Anspruch 20.

Gemäß der Erfindung werden zwei verschiedene grafische Modi vorgesehen, um den selben Restzahnbereich und/oder die selbe Zahnersatzteilversorgung anzuzeigen. Durch das Vorsehen von mindestens zwei verschiedenen grafischen Modi ist es möglich, jeweils verschiedene relevante Aspekte der Darstellung hervorzuheben, die mit einem anderen Modus nicht möglich oder nicht so gut hervorhebbar sind.

So ist es beispielsweise in einem Modus möglich, technische Hilfsinformation, wie Hilfslinien, Flächeneinteilungen, Falschfarbendarstellungen etc. vorzusehen, wohingegen der andere Modus daraufhin optimiert sein kann, eine möglichst naturgetreue Darstellung des vorgesehenen Endergebnisses der Versorgung darzustellen.

Der zweite Modus kann sich so z. B. durch eine höhere Bildauflösung, eine größere Anzahl von möglichen Farben, die Darstellung von Schattenwürfen, Spiegeleffekten oder Reflexen, Transparenzen oder Nebeleffekten von dem anderen Modus unterscheiden. Auch eine größere Anzahl von simulierten Lichtquellen oder von diffusen bzw. nichtdiffusen Lichtquellen sowie eine zentralperspektivische Darstellung oder eine farbrealistische Darstellung von verschiedenen Bereichen kann den zweiten Modus von dem ersten Modus unterscheiden.

Zwischen den beiden grafischen Modi kann hin und her gewechselt werden. Hierzu können entsprechende Schaltflächen-, Tastatur- oder Mausklick-Kombinationen, Spracheingaben oder sonstige Bedienmöglichkeiten eines Computers vorgesehen sein.

Bei der Änderung des grafischen Modi wird vorzugsweise derjenige Teil des Restzahnbereichs bzw. der Zahnersatzteilversorgung und/oder der Blickpunkt bzw. die Blickrichtung nicht verändert. Der Bildausschnitt bleibt vorzugsweise identisch, lediglich die Art der Darstellung wird geändert.

Der erste Modus kann sich von dem zweiten grafischen Modus auch dadurch unterscheiden, dass die Darstellung von Falschfarben für die Darstellung von technischen Informationen möglich ist oder, dass Hilfslinien, Hilfsflächen, simulierte Linien, Schichten oder Spalten möglich sind. Auch kann bei dem ersten Modus, im Gegensatz zum zweiten Modus, ein Hinterschnitt grafisch durch Falschfarben oder sonstige grafische Hervorhebungen kenntlich gemacht werden. Auch kann der erste Modus nur eine einzelne simulierte Lichtquelle (diffus oder direkt strahlend) aufweisen.

Weiterhin können beispielsweise maximal fünf verschiedene Grundfarben vorgesehen sein, die auch in verschiedenen Helligkeitsstufen darstellbar sind. Auch kann diese Ansicht im Gegensatz zum zweiten Modus eine isometrische Darstellung aufweisen, da diese zum Auslesen von technischer Information besser geeignet ist.

Der zweite Modus dient also somit im Wesentlichen dazu, eine Darstellungsart zur Verfügung zu stellen, in der insbesondere ästhetische Aspekte der geplanten Zahnersatzteilversorgung gut erkennbar gemacht werden, wohingegen der erste Modus dazu dient, technische Information erkennbar zu machen.

Die Darstellung im ersten grafischen Modus kann in einem separaten Fenster auf dem Bildschirm erfolgen und die Darstellung im zweiten grafischen Modus auf einem separaten zweiten Fenster. Beide Darstellungen können auch über- oder nebeneinander in ein und demselben Fenster angezeigt werden. Die verschiedenen Darstellungsarten können auch durch andere Medien, als auf einem Bildschirm, ausgegeben werden. Dies kann ein Drucker sein oder das Versenden von entsprechenden Grafikdateien. Auch eine Speicherung auf Datenträgern (CD, DVD, Memory-Stick oder andere elektronische Speicherelement, Diskette) und die Versendung derselben ist möglich.

Auch können gleichzeitig verschiedene Ansichten (Ausschnitte, Blickwinkel etc.) des Restzahnbereichs und/oder der Zahnersatzversorgung möglich sein, wobei für jede Ansicht der erste und/oder zweite grafische Modus wählbar ist.

Die Daten für die Darstellungen werden vorzugsweise bei einem Zahntechniker erzeugt. Von dort können die Daten für die Darstellung an einen Patienten und/oder einen Zahnarzt, also allgemein an einen anderen Ort, übersandt werden. Dort kann die Darstellung in dem zweiten grafischen Modus, aber auch in dem ersten grafischen Modus, erfolgen. Aufgrund der Darstellung kann ein Zahnarzt oder ein Patient die geplante Zahnersatzteilversorgung genehmigen oder aber auch Änderungen wünschen. So kann ein Patient z.B. eine andere Farbgestaltung oder eine andere Form oder Konstruktion einer Zahnersatzteilversorgung wünschen. Hierbei ist es auch von Vorteil, wenn weitere Information, die nicht Bildinformation ist, mit übertragen wird und dargestellt wird. Diese weitere Information kann z.B. der Preis, die Angabe des Materials, Versorgungstyp, Lieferzeit, Anzahl der nötigen Besuche beim Zahnarzt, Gewährleistungsinformation, Patientenidentifikationsdaten, Planungsidentifikationsdaten etc. umfassen.

Daten eines Datensatzes, der die geplante Zahnersatzteilversorgung speichert, können durch Bearbeitung in dem ersten und/oder zweiten grafischen Modus erfolgen. Nach oder während einer Veränderung der Daten der Zahnersatzteilversorgungen werden die neuen (modifizierten Daten) in beiden oder nur dem jeweils gewählten Modus dargestellt.

Erfindungsgemäß können weiterhin ein entsprechendes computerlesbares Medium zur Durchführung eines solchen Verfahrens, ein Computer mit Mitteln für die Durchführung eines solchen Verfahrens oder ein Verfahren zum elektronischen Versenden einer Datei mit Instruktionen für einen Computer zur Durchführung eines solchen Verfahrens, vorgesehen sein.

Eine bevorzugte Ausführungsform der Erfindung ist in der beiliegenden Figur dargestellt. Hierbei zeigt diese Figur einen Restzahnbereich mit einer geplanten Zahnersatzteilversorgung in zwei verschiedenen grafischen Modi.

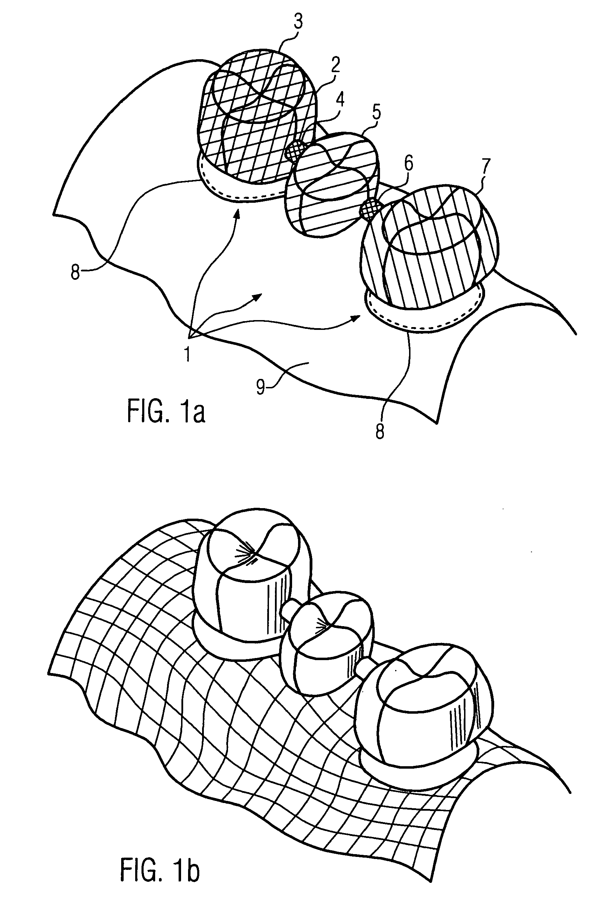

In Figur 1 a und 1b sind genau die gleichen Bereiche und Ansichten ein und desselben Restzahnbereichs 1 und der geplanten Zahnersatzteilversorgung 2 gezeigt. Das Zahnersatzteil 2 ist hier eine Brücke, die auf zwei Zahnstümpfe aufgesetzt ist und dazwischen ein Pontic 5 aufweist, das über Stege 4, 6 mit den Elementen 3, 7, die auf den Zahnstümpfen aufgesetzt sind, verbunden ist.

In Figur 1b ist die Darstellung aus der Figur 1 a dahingehend abgewandelt, dass verschiedene Flächen verschieden schraffiert bzw. gestrichelt sind, um anzudeuten, dass der grafische Modus in Figur 1b ein anderer als der in Figur 1 a ist.

In Figur 1 a ist beispielsweise das Käppchen 3 gekreuzt schraffiert dargestellt, was andeuten soll, dass dieses in beispielsweise einer anderen Farbe dargestellt werden kann, als der Steg 4 oder das Pontic 5. Diese sind jeweils in einer anderen Schraffur dargestellt, was andeuten soll, dass diese in einer anderen Farbe dargestellt werden können. Die Darstellung in den verschiedenen Farben soll beispielsweise technische Informationen darstellen, die erleichtert, die verschiedenen Elemente als solche identifizieren zu können.

Weiterhin ist beispielsweise eine Hilfslinie 8 eingezeichnet, die den Rand des Zahnfleischbereichs 9 erkenntlich macht.

In Figur 1b ist der Zahnfleischbereich 9 in einer quadratischen Schraffur dargestellt, was andeuten soll, dass dieser Bereich beispielsweise in realistischen Fleischfarben dargestellt wird. Das Zahnersatzteil 2 hingegen ist mit einer durch Strichelung angedeuteten Schattendarstellung dargestellt. Etwaige Falschfarben oder Hilfslinien sind hier nicht dargestellt. Die Darstellung in Figur 1b dient somit dazu, einen (photo-) realistischen Eindruck der geplanten Zahnersatzteilversorgung zu erhalten, um ästhetische Aspekte der Zahnersatzteilversorgung prüfen zu können, wohingegen die Darstellung in Figur 1 a dazu dient, technische Informationen darzustellen, beispielsweise, um eine Modifikation des Zahnersatzteils zu erleichtern oder beispielsweise Wandstärken oder andere konstruktive Gegebenheiten zu überprüfen.

In beiden Ansichten kann (z. B. mit einer Maus) eine Bearbeitung der Daten erfolgen. Die so modifizierten Daten können in beiden Darstellungen neu dargestellt werden, so dass Änderungen der Daten in der einen, der anderen oder beiden Darstellungen nachvollzogen werden können.

Die photorealistische Darstellung kann an einem anderen Ort dargestellt werden, als an dem sie erzeugt wird oder an dem eine Darstellung im ersten grafischen Modus erfolgt. So kann z.B. ein Zahntechniker einen Entwurf einer Zahnersatzteilversorgung erstellen und dieser einem Patienten und/oder Zahntechniker zur Begutachtung geschickt werden. Dieser kann dann z.B. Änderungen vorschlagen oder die geplante Behandlung genehmigen. Aufgrund von einem oder mehreren Änderungswünschen kann die geplante Zahnersatzteilversorgung modifiziert und erneut zur Darstellung für den Patienten und/oder Zahnarzt verschickt werden. Die Darstellung beim Patienten oder Zahnarzt kann auf einem Bildschirm oder einem Ausdruck erfolgen. Die Änderungen können Änderungen von Form und/oder Farbe und/oder Material einer geplanten Zahnersatzteilversorgung sein.

Auch ist es möglich mehrere geplante Zahnersatzteilversorgungen darzustellen, beispielsweise Versorgungen mit verschiedenen Materialien und/oder Konstruktionen. So können verschiedene Varianten mit ihren jeweiligen Vor- und/oder Nachteilen zur Begutachtung verschickt werden. Zu jeder der mehreren Versorgungen kann auch weitere Information, die keine Bildinformation ist, mit dargestellt bzw. verschickt werden, wie etwa eine Information zu Preis, Information zu Material, Versorgungstyp (Brücke, Krone, Implantat, etc.), Lieferzeit, Anzahl der notwendigen Zahnarztbesuche für eine entsprechende Behandlung, Gewährleistungsinformation, Patientenidentifikationsdaten, Planungsidentifikationsdaten oder Ähnliches. Die Darstellung bei einem Zahnarzt kann auch für einen Patienten erfolgen, etwa bei einem Zahnarztbesuch des Patienten. Die Darstellung für einen Patienten kann jedoch auch unabhängig von einem Zahnarzt, beispielsweise bei dem Patienten zu Hause oder an seiner Arbeitsstätte erfolgen.

Auch allein die Darstellung des Restzahnbereichs ohne eine geplante Zahnersatzteilversorgung kann vorgesehen sein, etwa um Besonderheiten des Restzahnbereichs darzustellen.

Auch die Darstellung im ersten Modus beim Patienten und/oder Zahnarzt (also an einem anderen Ort) kann erfolgen, beispielsweise um bestimmte technische Aspekte zu erläutern.

## Patentansprüche

1. Verfahren zum Darstellen von Restzahnbereichen (1) und/oder geplanten Zahnersatzteilversorgungen (2) mit den Schritten:
- Darstellen des Restzahnbereichs (1) und/oder der Zahnersatzteilversorgung (2) in einem ersten grafischen Modus und
- Darstellen desselben Restzahnbereichs (1) und/oder der Zahnersatzteilversorgung (2) in einem zweiten grafischen Modus, der verschieden ist von dem ersten grafischen Modus.

2. Verfahren nach Anspruch 1, **dadurch gekennzeichnet, dass** der zweite grafische Modus sich von dem ersten unterscheidet durch einen, zwei, drei, vier oder mehr der folgenden Punkte:
- eine höhere Bildauflösung
- eine größere Anzahl von möglichen Farben
- die Darstellung von Schattenwurf
- die Darstellung von Spiegeleffekten und/oder Reflexen
- die Darstellung von Transparenzen
- die Darstellung von Nebeleffekten
- eine größere Anzahl von simulierten Lichtquellen
- farbrealistische Darstellung von verschiedenen Bereichen, wie etwa Zahnfleisch, Zahnmaterial oder Zahnersatzteilen
- zentralperspektivische Darstellung mit einem, zwei oder drei Fluchtpunkten.

3. Verfahren nach einem der Ansprüche 1 oder 2, **dadurch gekennzeichnet, dass** zwischen den beiden grafischen Modi hin und her gewechselt werden kann.

4. Verfahren nach einem der Ansprüche 1 bis 3, **dadurch gekennzeichnet, dass** bei dem Wechsel des grafischen Modus der dargestellte Teil des Restzahnbereichs (1) und/oder der Zahnersatzteilversorgung (2) und/oder der Blickpunkt und/oder die Blickrichtung des Restzahnbereichs (1) und/oder der Zahnersatzteilversorgung (2) nicht verändert wird.

5. Verfahren nach einem der Ansprüche 1 bis 4, **dadurch gekennzeichnet, dass** sich der erste grafische Modus von dem zweiten grafischen Modus unterscheidet durch einen, zwei drei, vier oder mehr der folgenden Punkte:
- Darstellung von Falschfarben für die Darstellung von technischen Informationen, wie etwa Hinterschneidungstiefen, Bruchfestigkeiten, Wandstärken oder Ähnlichem
- Darstellung von Falschfarben für die Unterscheidung von verschiedenen Bereichen, wie etwa Zahnfleisch, Zahnmaterial oder Zahnersatzteilen
- Darstellung von Hilfslinien oder Hilfsflächen, wie etwa von Präparationslinien, Friktionslinien, simulierten Stumpflackschichten, simulierten Zementspalten
- grafische Hervorhebung von Hinterschnitten
- eine simulierte Lichtquelle
- maximal fünf verschiedene Grundfarben, die jedoch in verschiedenen Helligkeitsstufen dargestellt werden können
- isometrische Darstellung.

6. Verfahren nach einem der Ansprüche 1 bis 5, **dadurch gekennzeichnet, dass** eine Darstellung im ersten grafischen Modus in einem ersten Fenster erfolgt und gleichzeitig eine Darstellung im zweiten grafischen Modus in einem zweiten Fenster erfolgt.

7. Verfahren nach einem der Ansprüche 1 bis 6, **dadurch gekennzeichnet, dass** die Darstellung im ersten und/oder zweiten grafischen Modus auf einem Drucker ausgedruckt wird und/oder per Email, Instant-Messaging, Internet, Webseite, oder auf einem Datenträger wie Diskette, einem elektronischen Speicherelement, CD oder DVD verschickt wird, wobei das Drucken oder Verschicken vorzugsweise so erfolgt, dass nur die Darstellung im zweiten grafischen Modus verwendet wird oder die gleichzeitige Darstellung im ersten grafischen Modus und im zweiten grafischen Modus separat, aber auf einer Seite oder in einem Dokument.

8. Verfahren nach einem der Ansprüche 1 bis 7, **dadurch gekennzeichnet, dass** verschiedene Ansichten des Restzahnbereichs und/oder der Zahnersatzteilversorgungen gleichzeitig möglich sind, wobei für jede Ansicht der erste oder der zweite grafische Modus wählbar sind.

9. Verfahren nach einem der Ansprüche 1 bis 8, **dadurch gekennzeichnet, dass** die Darstellung des zweiten grafischen Modus zusätzlich oder alternativ an einem anderen Ort als demjenigen Ort der Darstellung des ersten grafischen Modus erfolgt.

10. Verfahren nach einem der Ansprüche 1 bis 9, **dadurch gekennzeichnet, dass** die Darstellung im ersten und/oder zweiten grafischen Modus bei einem Zahnarzt oder Patienten erfolgt, während die Darstellung im ersten grafischen Modus vorzugsweise bei einem Zahntechniker erfolgt.

11. Verfahren nach einem der Ansprüche 9 oder 10, **dadurch gekennzeichnet, dass** ein Patient oder ein Zahnarzt aufgrund der Darstellung im ersten und/oder zweiten grafischen Modus einen Änderungswunsch oder sein Einverständnis mit der geplanten Zahnersatzteilversorgung äußert und dieser/dieses vorzugsweise an einen Ort der Darstellung im ersten grafischen Modus und/oder an einen Zahntechniker übermittelt wird.

12. Verfahren zum Darstellen von Restzahnbereichen und/oder geplanten Zahnersatzteilversorgungen mit dem Schritt:
- Darstellen des Restzahnbereichs und/oder der Zahnersatzteilversorgung in einem grafischen Modus, der einen, zwei, drei, vier oder mehr der folgenden Punkte aufweist:
- eine photorealistische Darstellung
- eine Bildauflösung von höher als 300 dpi
- mindestens eine 24 bit- Farbtiefe
- die Darstellung von Schattenwurf
- die Darstellung von Spiegeleffekten und/oder Reflexen
- die Darstellung von Transparenzen
- die Darstellung von Nebeleffekten
- mindestens zwei, drei, vier oder mehr simulierte Lichtquellen
- farbrealistische Darstellung von verschiedenen Bereichen, wie etwa Zahnfleisch, Zahnmaterial oder Zahnersatzteilen.

13. Verfahren nach Anspruch 12, **dadurch gekennzeichnet, dass** die Darstellung auf einem Drucker ausgedruckt wird und/oder per Email, Instant-Messaging, Internet, Webseite oder auf einem Datenträger wie etwa einer Diskette, einem elektronischen Speicherelement, einer CD oder einer DVD verschickt wird.

14. Verfahren nach Anspruch 12 oder 13, **dadurch gekennzeichnet, dass** die Daten für die Darstellung an einen anderen Ort geschickt werden und dort dargestellt werden, wobei die Darstellung vorzugsweise für oder bei einen Patienten und/oder einen Zahnarzt erfolgt.

15. Verfahren nach Anspruch 14, **dadurch gekennzeichnet, dass** aufgrund der Darstellung ein Anwender wie etwa ein Patient oder ein Zahnarzt einen Änderungswunsch oder sein Einverständnis mit der Zahnersatzteilversorgung äußert und dieser/dieses vorzugsweise an den Ort übermittelt wird, von dem die Daten für die Darstellung stammen, wie etwa der Ort eines Zahntechnikers.

16. Verfahren nach einem der Ansprüche 1 bis 15, **dadurch gekennzeichnet, dass** die Daten des dargestellten Restzahnbereichs und/oder der geplanten Zahnersatzteilversorgung modifiziert werden und so modifiziert neu dargestellt werden.

17. Verfahren nach einem der Ansprüche 1 bis 16, **dadurch gekennzeichnet, dass** die Darstellung von mehreren Zahnersatzteilversorgungen erfolgt.

18. Computerlesbares Medium mit Instruktionen, die in einen Computer eingeladen, diesen veranlassen, ein Verfahren nach einem der Ansprüche 1 bis 17 auszuführen.

19. Ein Computer oder mehrere Computer mit Mitteln, ein Verfahren nach einem der Ansprüche 1 bis 17 auszuführen.

20. Verfahren mit dem Schritt des elektronischen Versendens einer oder mehrerer Dateien, beispielsweise über das Internet, wobei die Datei Instruktionen enthält, die in einen oder mehrere Computer eingeladen, diesen/diese veranlassen, ein Verfahren nach einem der Ansprüche 1 bis 17 auszuführen.
